# EUROPEAN PATENT APPLICATION

(11) **EP 3 815 624 A1**
(43) Date of publication of application: **05.05.2021**
(21) Application number: 19206358.4
(22) Date of filing: 30.10.2019
(51) Int. Cl.: A61B 17/02, A61B 17/42

(54) **A SURGICAL ACCESS SYSTEM**

(71) Applicant: National University of Ireland Galway, Galway (IE)
(72) Inventor: Maher, Padraig, Gort, Co. Galway (IE); McCann, Barry, Moycullen, Co. Galway (IE); Maher, Marie-Therese, Gort, Co. Galway (IE)
(74) Representative: Purdylucey Intellectual Property

(57) **Abstract**

A surgical access system adapted to facilitate access to a surgical site through an incision in a patient's body by opening the incision is described. The system comprises a supporting ring (2), at least two adjustable saddle-shaped retractors (3, 17); and coupling elements (20) configured for retro-fitting the adjustable saddle-shaped retractors to the supporting ring in an outwardly facing orientation. The supporting ring comprises a fixed saddle-shaped retractor (7) fixed to the supporting ring and an outwardly projecting handle (8) fixed to the supporting ring. The system is typically configured for use in a caesarean section procedure, especially in an obese patient, where the supporting ring is dimensioned to allow delivery of a neonate through the ring, and the fixed saddle-shaped retractor is a pelvic-region retractor.

## Description

### Field of the Invention

The present invention relates to a surgical access system, in particular, a surgical access system for use in a caesarean section procedure. Also contemplated are methods of facilitating access to a surgical site, in particular, during a caesarean section.

### Background to the Invention

Management of the abdominal tissue and gaining sufficient access to the abdomen & uterus during c-sections currently involves using one of, or a combination of, the following methods:
Metal retractors, including hook & prong retractors, Balfour & Collin retractors with centre blades, bladder protection retractors such as Doyens Retractors, Bladder Blades, etc. Although these are effective for gaining visibility and access where required in the surgery, they require additional hands/assistants in order to hold each one, they cannot be left in place during delivery of the baby and depending on the shape of the retractor itself can induce local trauma to the abdominal tissue.

Plastic double-ring disposable retractors consisting of an inner & outer ring held together with a hammock of clear flexible film which protects the wound edge. The primary competitors in this area are the Alexis O Retractor (by Applied Medical), SurgiSleeve (by Medtronic) and OB/Mobius (by Cooper Surgical). All of these competitors have addressed the need in a similar way by providing a double ring & hammock design. However, these products are adapted from other surgeries so none of them address the particular clinical & ergonomic needs of caesarean surgery. These products are easy to use, keep the wound symmetrically held open and are safe to deliver a baby through, however they lose their effectiveness when dealing with overweight or obese patients because they are not capable of effectively holding back a dense wall of abdominal tissue, or dealing with an overhanging pannus.

Additional hands; assistance is sought from medical staff to use their hands to pull back & hold the abdominal tissue out of the way of the performing obstetrician, as required. This can provide immediate effectiveness for the obstetrician but costs time & money for additional staff, causes physical strain & fatigue for those staff, and proportionately increases the risk of transfer of bacteria from the patients' skin into the wound. In addition, the assistant (who is generally on the opposite side of the table to the lead), is a more effective assistant - their hands are free to be more effective as opposed to spending time holding up heavy flesh.

In caesarean cases for patients with very high BMI, a combination of the above techniques may be required along with some extreme measures such as taping of the pannus to keep it out of the working space of the obstetrician.

US4421107 describes a surgical retractor comprising a supporting ring and a plurality of radially adjustable retractor paddles attached to the ring at defined positions are the ring. During use, the retractor is placed over the incision, and the paddles engage the incision before being radially retracted to open the incision while attached to the ring. The circumferential position of the paddles on the ring cannot be adjusted during the surgery.

It is an object of the invention to overcome at least one of the above-referenced problems.

### Summary of the Invention

The Applicant has addressed the problems of the prior art by providing a surgical retractor that has one or more detachable tissue cupping retractors that can be hand-held and used to engage an incision to cup a section of tissue, and then be manually retracted and attached to a supporting ring in a tissue retracting position where it is retained in position unless further adjusted while attached to the supporting ring. Compared with the system of the prior art, this allows freedom to manually articulate the retractors during engagement with the incision and opening of the incision, and allows the retractors to be attached to the supporting ring once the user is satisfied that they are in the correct position and held in place by the supporting ring for the remainder of the procedure, or further retracted as required. In a preferred embodiment, coupling elements are provided for easy attachment to the supporting ring during surgery that are self-locking, i.e. once attached to the ring, they retain their position on the ring unless adjusted by the user, and/or configured to allow radial or circumferential adjustment of the retractor(s) on the supporting ring. In addition, the use of saddle-shaped retractor is especially suitable as the retractor mimics a human's hand and spreads the retracting forces along a section of the incision.

In a first aspect, the invention provides a surgical access system adapted to facilitate access to a surgical site through an incision in a patient's body by opening the incision, comprising:
a supporting ring;
a detachable saddle-shaped retractor configured for cupping and manually retracting a section of tissue at the incision and attachment to the supporting ring in a tissue-retracting position.

The system generally comprises a coupling element configured for retro-fitting the adjustable saddle-shaped retractor to the supporting ring after the section of tissue has been retracted and retaining the retractor in the tissue-retracting position. In one embodiment, the coupling element is separate from the ring and the retractor and configured for attachment to both the retractor and ring. In one embodiment, the coupling element is configured to snap-fit to the ring - this allow freedom in positioning the coupling element in multiple positions along the circumference of the ring.

The saddle-shaped retractor is shaped for handling during manual retraction and cupping the tissue, and typically comprises an upper wall, lower wall, and rear wall (suitable for clasping by a human hand). In one embodiment, the retractor has a U-shaped profile. In one embodiment, the rear wall has convex profile to follow the curvature of an opened incision.

The coupling element is generally configured for coupling the saddle-shaped retractor to the supporting ring and maintaining the position of the retractor on the ring when the retractor is released by the user (i.e. self-locking).

In another aspect, the invention provides a surgical access system adapted to facilitate access to a surgical site through an incision in a patient's body by opening the incision, comprising:
a supporting ring;
at least two adjustable retractors configured to engage a section of the incision; and
a coupling element configured for coupling each adjustable saddle-shaped retractor to the supporting ring in an outwardly facing orientation.
wherein the coupling elements is configured to allow radial or circumferential adjustment (or both radial and circumferential adjustment) of the adjustable tissue retracting paddle while attached to the supporting ring from a first tissue-retraction position to a second tissue retraction position.

In another aspect, the invention provides a surgical access system adapted to facilitate access to a surgical site through an incision in a patient's body by opening the incision, comprising:
a supporting ring; and
at least two tissue retractors coupled to the supporting ring;
wherein at least one of the retractors comprises an upwardly depending panniculus deflecting lip.

In another aspect, the invention provides a saddle-shaped retractor for use in a surgical access system of the invention, having a U-shaped tissue-cupping profile defined by an upper wall, lower wall and rear wall, and an elongated slot in the upper wall.

In one embodiment, the system is configured for accessing a surgical site through a caesarean incision in a woman's (or higher mammals) abdomen. In such an embodiment, the supporting ring is generally dimensioned to allow delivery of a newborn through the supporting ring.

In one embodiment, the supporting ring comprises a saddle-shaped retractor fixed to the supporting ring. In one embodiment, the retractor is a pelvic region retractor (often referred to as a "Doyen" Retractor). This arrangement allows a surgeon use the fixed retractor to secure the supporting ring to the incision, and then use the adjustable retractor(s) to engage the incision and retract tissue to open the incision before they are attached to the supporting ring in an incision-opening position and retained in position. When the system is configured for use with a Caesarean section, the fixed retractor will generally be a pelvic region retractor, configured to cover the woman's bladder. In another embodiment, the fixed retractor has a panniculus-deflecting lip.

In one embodiment, the supporting ring comprises an outwardly projecting handle fixed to the supporting ring (generally adjacent the fixed retractor). Generally, the handle is projects upwardly and outwardly of the supporting ring. This facilitates initial positioning of the supporting ring.

In one embodiment, the coupling element is configured to allow adjustment of the adjustable tissue retracting paddle on the supporting ring from a first retraction position to a second retraction position. In one embodiment, the coupling element is self-locking (i.e. it is configured to lock the retractor in position on the supporting ring when the retractor is coupled to the ring and released by the user).

In one embodiment, the coupling element is configured to allow radial or circumferential adjustment (or both radial and circumferential adjustment) of the adjustable tissue retracting paddle on the supporting ring.

The term "radial" as used herein should be understood to mean movement of the retractor to open or close the incision, i.e. from a position within the ring towards a periphery of the ring, or *vica versa.* The movement does not have to be exactly radial. The provision of a coupling element that allows easy radial adjustment of the position of the retractor with respect to the supporting ring, provides the flexibility to allow controlled further opening (or closing) of the incision after the retractor has been attached to the ring.

In one embodiment, the retractor and coupling element are configured for a radial sliding engagement. In one embodiment, the adjustable saddle-shaped retractor comprises a radial slot, and the coupling element comprises an element, for example a lug, configured for sliding engagement in the slot. A brake may be provided that is actuatable to lock the adjustable tissue retracting paddle to the coupling element. Preferably, the lug and slot are self-locking (for example, when the slot and coupling element may be configured to lock in position when the retractor is released by the user). In one embodiment, the radial slot is a re-entrant slot.

In another embodiment, the retractor may comprise means for snap-fit engagement with the coupling element. In one embodiment, the retractor may comprise a plurality of spaced apart snap-fit engagement means.

In one embodiment, the coupling element is configured for movement along the supporting ring from a first circumferential position to a second circumferential position.

In one embodiment, the coupling element comprises a carriage that is moveable along the ring and optionally comprises brake means for fixing the carriage in a desired circumferential position along the ring.

In one embodiment, the system comprises at least two adjustable saddle-shaped retractors and associated coupling elements.

In one embodiment, the system comprises at least 3, 4, 5, 6, 7, 8, 9 or 10 adjustable saddle-shaped retractors and associated coupling elements.

In one embodiment, at least one of the adjustable saddle-shaped retractors is configured for adjustment of the height of the retractor. The term "height" as used herein refers to the distance between the upper and lower walls of the retractors. This arrangement allows the user to adjust the retractor (prior to or after engagement with the tissue) to allow a closer fit between the tissue and the retractor. It is especially useful in the context of abdominal surgery where the thickness of the abdominal wall may vary widely from patient to patient.

In one embodiment, the rear wall of the adjustable saddle-shaped retractor has an outwardly curved (i.e. convex) profile. This allows the rear wall to follow the curvature when the incision has been opened.

In one embodiment, a curvature of the convex rear wall of the adjustable saddle-shaped retractor approximately matches the curvature of the supporting ring.

In one embodiment, the upper and lower walls of the adjustable saddle-shaped retractor are splayed outwardly.

In one embodiment, the adjustable tissue retracting paddle has a U-shaped profile.

In one embodiment, an upper wall of the adjustable tissue retracting paddle comprises a generally trapezoid shape.

In one embodiment, the supporting ring and/or tissue retracting paddles are formed from a polymer. In one embodiment, the supporting ring and/or tissue retracting paddles are formed by injection moulding, 3-D printing or vacuum casting.

In one embodiment, the supporting ring and adjustable saddle-shaped retractors are configured to facilitate access to a surgical site during a caesarean section procedure through a caesarean incision in a woman's abdomen.

In one embodiment, a free end of the upper wall of at least one (and typically 2, 3 or 4) of the adjustable saddle-shaped retractors comprises an upwardly depending panniculus deflecting lip.

In one embodiment, the system comprises a plurality of adjustable saddle-shaped retractors including at least one (and preferably at least two or three) comprising an upwardly depending panniculus deflecting lip and at least one without an upwardly depending panniculus deflecting lip.

In one embodiment, the supporting ring and/or retractor is configured to emit light. For example, it may include one or more lights. In one embodiment, the lights are positioned to direct light into the incision. In one embodiment, the ring comprises a deflector configured to deflect the light into the incision. In one embodiment, the lights are UV-lights configured to have a bacteriocidal effect.

In one embodiment, the retractors have a smooth surface configured to prevent the buildup or blood or clots on the surface of the retractors.

In one embodiment, at least part of the surface of the retractors comprises a coating of hydrophilic material configured to become slippery when in contact with body fluids and thereby facilitate ease of passage of the neonate during delivery.

In one embodiment, the retractor paddles comprise portions of semi-rigid polymer and portions of soft & flexible thermoplastic elastomer. This allows the retractors have structural rigidity to enable them to hold an incision open yet provide a patients interface that is soft and flexible.

In one embodiment, at least a part of the surface of the retractor and/or supporting ring comprises an anti-microbial surface. Such surfaces comprise materials that exhibit anti-microbial properties (i.e. nanoparticulate silver, etc).

In one embodiment, the system is disposable (i.e. it is configured for a single use).

In another aspect, the invention provides a method of facilitating access to a surgical site through an incision in a patient's body by opening the incision, comprising the steps of:
inserting a first adjustable saddle-shaped retractor into the incision to cup a first section of tissue;
attaching the first adjustable saddle-shaped retractor to a supporting ring while it is cupping the first section of tissue;
inserting a second adjustable saddle-shaped retractor into the incision to cup a second section of tissue; and
attaching the second tissue retracting paddle to the supporting ring while it is cupping the second section of tissue to hold open the incision.

In one embodiment, the incision is a caesarean incision and the tissue comprises abdominal tissue, and in which the supporting ring is dimensioned to allow delivery of a neonate through the ring.

In one embodiment, the supporting ring comprises a saddle-shaped retractor fixed to the ring, in which the method comprises an initial step of inserting the fixed saddle-shaped retractor into the incision to cup a section of abdominal tissue. In one embodiment, the fixed retractor is a pelvic-region retractor and the method comprises inserting the retractor into the incision to cup a section of abdominal tissue above the subject's bladder.

In one embodiment, the method comprises a further step of adjusting the radial position of the first or second tissue retracting paddle with respect to the supporting ring while it is attached to the supporting ring.

In one embodiment, the method comprises a further step of adjusting the circumferential position of the first or second tissue retracting paddle with respect to the supporting ring while it is attached to the supporting ring.

In one embodiment, the method comprises additional steps of inserting a third adjustable saddle-shaped retractor into the incision and cupping a third section of abdominal tissue with the retractor, and attaching the third retractor to the supporting ring to further open the incision.

In one embodiment, the method comprises a step of adjusting a height of at least one of the adjustable saddle shaped retractors prior to or after it has cupped a section of tissue.

In another aspect, the invention provides a method of facilitating access to a woman's womb through a caesarean incision in the woman's abdomen by opening the incision, comprising the steps of:
providing a supporting ring dimensioned to allow delivery of a neonate through the ring and comprising a saddle-shaped pelvic-region retractor fixed to the supporting ring;
inserting the fixed saddle-shaped pelvic-region retractor into the incision to cup a first section of abdominal tissue over the woman's bladder;
inserting a first adjustable saddle-shaped retractor into the incision to cup a second section of abdominal tissue;
attaching the first adjustable saddle-shaped retractor to the supporting ring at a position spaced apart from the fixed saddle-shaped pelvic-region retractor to open the incision;
inserting a second adjustable saddle-shaped retractor into the incision to cup a third section of abdominal tissue with the retractor; and
attaching the second adjustable tissue retracting paddle to the supporting ring to further open the incision.

In one embodiment, the method comprises additional steps of inserting a third adjustable saddle-shaped retractor into the incision and cupping a fourth section of abdominal tissue with the retractor and attaching the third adjustable saddle-shaped retractor to the supporting ring to further open the incision.

In one embodiment, the method comprises a further step of adjusting the radial position of at least one of the adjustable saddle-shaped retractors with respect to the supporting ring while it is attached to the supporting ring.

In one embodiment, the supporting ring comprises a coupling element that is configured to allow radial adjustment of the adjustable retractor on the supporting ring from a first retraction position to a second retraction position.

In one embodiment, the adjustable retractor comprises a radial slot, and the coupling element comprises a lug configured for sliding engagement in the slot and a brake that is actuatable to lock the adjustable tissue retracting paddle to the coupling element. In one embodiment, the brake is self-locking.

In one embodiment, the method comprises a further step of adjusting the circumferential position of at least one of the adjustable saddle-shaped retractors on the supporting ring, typically while it is attached to the supporting ring.

In one embodiment, the coupling element is configured for movement along the supporting ring from a first circumferential position to a second circumferential position, wherein the method comprises a step of circumferential adjustment of the retractor on the supporting ring.

In one embodiment, the coupling element comprises a carriage that is moveable along the ring and comprises brake means for fixing the carriage in a position along the ring.

In one embodiment, the method comprises a step of adjusting the height of the adjustable saddle-shaped retractor prior to or after it cups the tissue.

In one embodiment, an upper wall of at least one of the adjustable saddle-shaped retractors comprises an upwardly depending panniculus deflecting lip, wherein the upwardly depending lip deflects the woman's panniculus away from the incision when it is attached to the supporting ring.

Other aspects and preferred embodiments of the invention are defined and described in the other claims set out below.

### Brief Description of the Figures

**Figure 1** is a perspective view of a supporting ring forming part of a surgical access system according to one embodiment of the invention and having a fixed saddle-shaped retractor.
**Figure 2A and 2B** are perspective views of a first embodiment of an adjustable saddle-shaped retractor forming part of a surgical access system according to the invention.
**Figure 3A and 3B** are perspective views of a second embodiment of an adjustable saddle-shaped retractor forming part of a surgical access system according to the invention and having a panniculus-deflecting lip.
**Figure 4** is a perspective view of a coupling element forming part of a surgical system according to the invention.
**Figure 5** is a perspective view of a surgical system according to one embodiment of the invention with the retractors attached to the supporting ring in a partly retracted configu ration.
**Figure 6** is a perspective view of the surgical system of Figure 5 with the retractors shown in a fully retracted configuration suitable for providing access to the uterus and womb during a caesarean section procedure.
**Figure 7** is an illustration of the use of one embodiment of the surgical access system of the invention in a caesarean section procedure, in which:
**Figure 7A** is an illustration of a pregnant woman with a caesarean incision (to be shown on the figure) and a supporting ring forming part of surgical access system;
**Figure 7B** shows supporting ring placed on the woman's abdomen with the fixed saddle-shaped retraction paddle inserted into the caesarean incision;
**Figure 7C** shows the coupling elements placed on the supporting ring at the positions where the retractors are to be positioned;
**Figure 7D** shows two adjustable saddle-shaped retractors (each having a panniculus-deflecting lip), one of which has been attached to the coupling element, and one of which is about to be attached to the coupling element. Although not shown in this illustration, prior to attaching to the supporting ring, the retractors will be uniformly hand-guided by a surgeon to safely clasp the desired abdominal wall area and will have been hand-retracted to the positions shown, thereby creating the required opening of the incision which may be specific to each surgeon's requirements.
**Figure 7E** shows two adjustable saddle-shaped retractors attached to the coupling element.
**Figure 7F** shows a third adjustable saddle-shaped retractor (also having a panniculus-deflecting lip) attached to the supporting via a snap-fit coupling element, and the two retractors of Figs 9E radially adjusted so that the rear walls of all three retractors are flush (again, although not shown, the retractor will have engaged an upper face of the incision and been hand-retracted by the surgeon to the position shown before attachment to the supporting ring).
**Figure 7G** illustrates the radial adjustment of the three adjustable retractors with respect to the supporting ring while attached to the ring to increase the size of the incision opening.
**Figures 7H** **and** **7I** illustrate two additional adjustable saddle-shaped retractors attached to the supporting ring via coupling elements (again, although not shown, the two retractors will have engaged a side of the incision and hand-retracted by the surgeon to the position shown before attachment to the supporting ring).

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, age, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s) (for example, the reduction in accumulation of pathological levels of lysosomal enzymes). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an *effective amount* or a *therapeutically effective amount* of an agent defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure. Improvement may be observed in biological / molecular markers, clinical or observational improvements. In a preferred embodiment, the methods of the invention are applicable to humans, large racing animals (horses, camels, dogs), and domestic companion animals (cats and dogs).

In the context of treatment and effective amounts as defined above, the term *subject* (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, camels, bison, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human. As used herein, the term "equine" refers to mammals of the family *Equidae,* which includes horses, donkeys, asses, kiang and zebra. The system and method of the invention is primarily for use in human surgery but can be easily adapted for use with large mammals, such as equine, bovine, supine and porcine mammals.

"Supporting ring" refers to a supporting structure in the form of a ring, generally a closed ring, upon which tissue retractors can be attached and retained in a tissue-retracting position. The ring is generally circular, but may also be oval-shaped, elliptical or relux triangle shaped. It is generally dimensioned to be approximately the same size or slightly larger than the desired size of the opened incision. When the system is for use in a caesarean section procedure, the ring is dimensioned to allow delivery of a newborn baby through the ring and is generally circular or oval shaped.

"Saddle-shaped retractor" refers to a tissue retracting paddle that is shaped to cup a section of an incision in a patient's body in a manner similar to a human hand, and generally has an upper wall, lower wall and rear wall with a U-shaped profile. The distance between the upper and lower walls is referred to as the "height", and the length of the rear wall in contact with the incision is referred to herein as the "width". The rear wall may have a convex profile, to match the curvature of an opened incision. The retractor may be configured for height adjustment. In one embodiment, an upper wall of the retractor comprises an upwardly depending lip configured to deflect a patient's panniculus away from the incision when the retractor is attached to the supporting ring. This is especially suitable for use with abdominal incisions, and in particular caesarean sections in obese women. The retractor may incorporate a degree of resilient deformability to allow a user clasp tissue when held in the hand.

"Retro-fitting" as applied to the retractor means that the retractor is separate from the supporting ring and designed to be easily attached to the supporting ring during a surgical procedure while in a tissue-retracting position, typically by means of a coupling element. Various coupling elements for attaching a retractor to the supporting ring are described herein, including the use of coupling elements having a lug configured to slidably engage in a radial slot in the retractor. In one embodiment, the coupling element comprises an element configured to snap-fit to the supporting ring. Embodiments described herein include carriage-like coupling elements configured for snap-fit coupling with the supporting ring. In one embodiment, the carriage is configured to allow circumferential movement of the carriage on the ring, while preventing or inhibiting rotational movement of the carriage on the ring. In other embodiment, the retractor includes in integral coupling element configured for direct engagement with the supporting ring, for example a groove dimensioned and position to snap-fit to the supporting ring. A plurality of integral coupling elements may be provided on the retractor to allow attachment to the supporting ring at different radial positions with respect to the supporting ring.

The coupling element of the invention may be self-locking. "Self-locking" as applied to the coupling element means that the coupling element locks the retractor on the ring once it has been attached and released by the user. An example is the use of a lug and slot arrangement described below, where once the slot on the retractor has engaged the lug, the forces exerted on the retractor by the opened incision cause the lug and slot to lock in position and remain in position until the position of the retractor is adjusted by the user. Other methods of self-locking coupling elements could include ratchet and pinion type mechanisms, or snap-fit coupling elements configured for friction fitting the retractor and supporting ring in relative position.

"Outwardly facing direction" as applied to the disposition of the retractor and the supporting ring means that the retractor, when attached to the supporting ring, faces outwardly to cup and hold a section of the incision in an open configuration.

"Fixed saddle-shaped retractor" means a retractor that forms part of the supporting ring and is not configured for retrofitting to, or detachment from, the supporting ring during surgery. When the system is for caesarean section surgery, the fixed retractor is typically a retractor of the type designed to hold back the bladder during a caesarean section.

"Radial adjustment" as applied to the retractor or coupling element should be understood to mean movement of the retractor to open or close the incision, i.e. from a position within the ring towards a periphery of the ring, or *vica versa.* The movement does not have to be exactly radial. The provision of a coupling element that allows easy radial adjustment of the position of the retractor with respect to the supporting ring, provides the flexibility to allow the incision to be further opened (or closed) after the retractor has been attached to the ring.

"Circumferential adjustment" as applied to the coupling element means that the coupling element is configured to allow movement of the retractor between at least two circumferential positions along the supporting ring. In one embodiment the coupling elements comprises a carriage configured for sliding movement along the supporting ring and may include brake means for fixing the position of the coupling element with respect to the supporting ring or be self-locking when position and released by a user.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

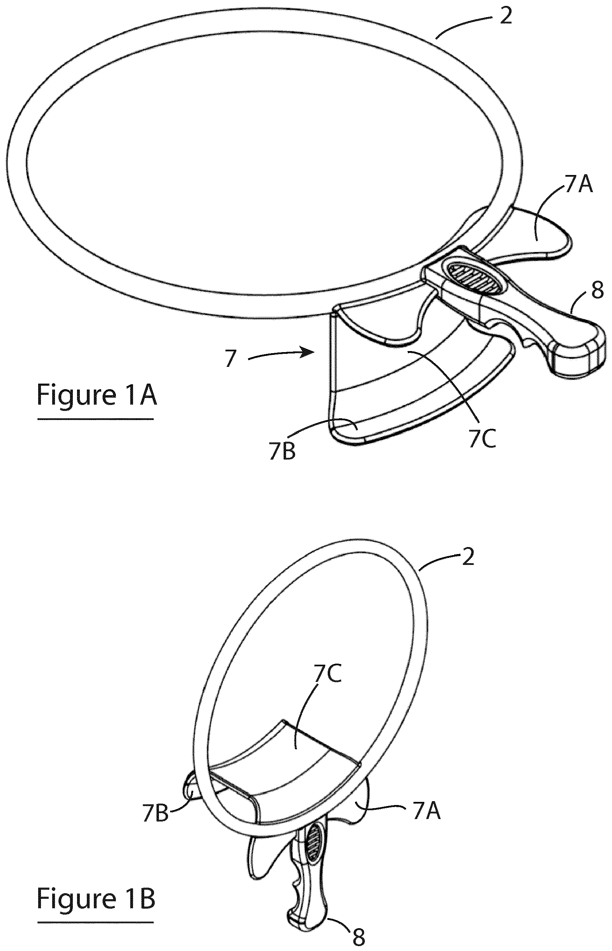

Referring to the drawings, and initially to Figures 1 to 4, there is illustrated a surgical access system of the invention, indicated generally by the reference numeral 1 and comprising a supporting ring 2, an adjustable saddle-shaped retractor 3, and a coupling element 4 for retro-fitting the adjustable retractor 3 to the supporting ring 2 in a desired position.

In more detail, and referring to Figure 1, the supporting ring 2 is circular and has a circumference sufficiently large to allow a newborn baby be delivered through the ring. Thus, this ring is slightly larger than a newborn infants head. The ring 2 includes a saddle-shaped retractor 7 fixed to the ring in an outwardly facing position, and a outwardly-projecting handle 8 disposed above the fixed retractor 7. The retractor 7 is generally U-shaped, and has an upper wall 7A, lower wall 7B, and rear wall 7C having a convex curvature that matches, and is aligned with, the curvature of the supporting ring 2. All of the corners 8 on the retractor are curved and do not include any sharp corners or edges.

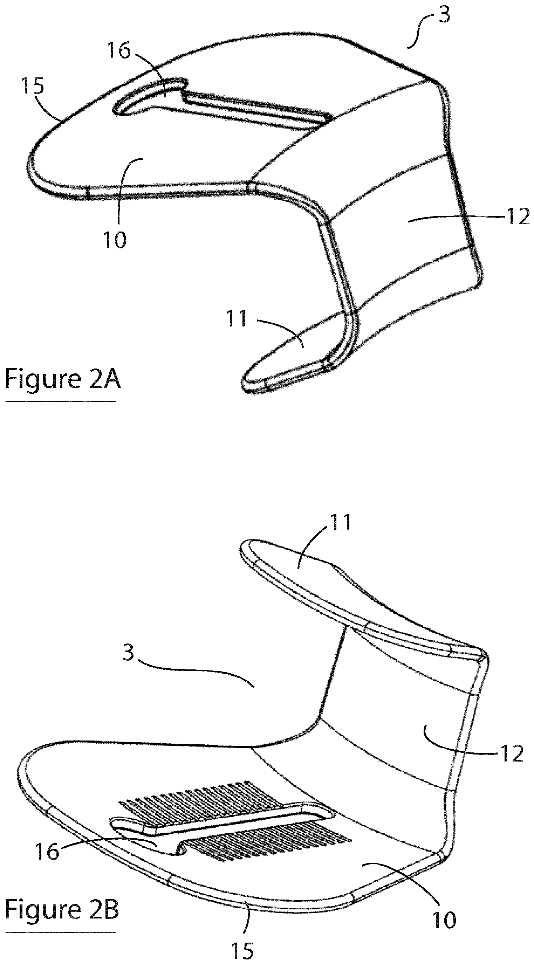

Referring to Figure 2, the adjustable saddle-shaped retractor 3 is shown in more detail, and includes an upper wall 10, lower wall 11, and rear wall 12 provided in a U-shapewith the upper and lower walls being slightly splayed outwardly. The rear wall 12 has a convex curvature. The upper wall 10 is longer than the lower wall 11 and has a trapezoid shape that widens from the rear wall 12 to a free end 15, and includes an elongated re-entrant slot 16.

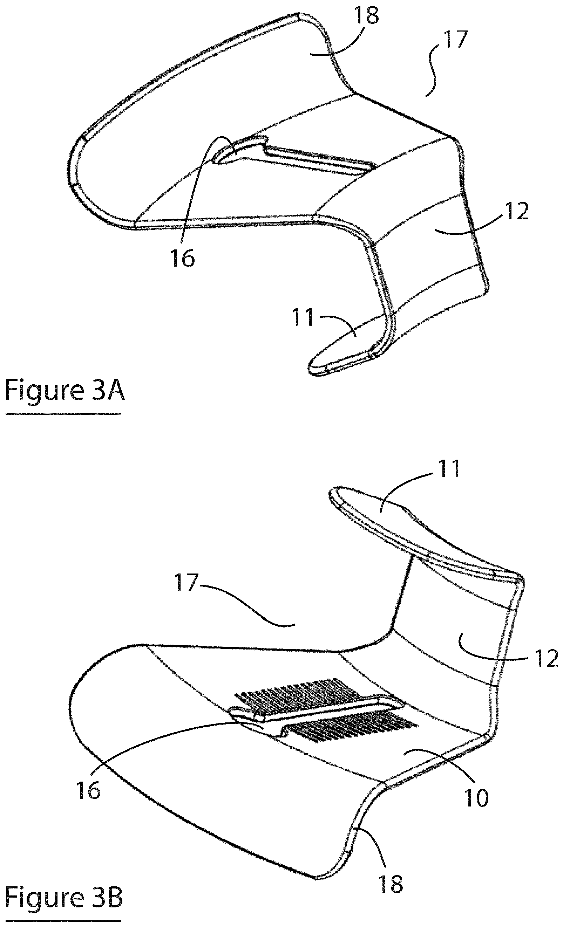

Referring to Figure 3, a modified adjustable saddle-shaped retractor 17 is illustrated in which parts identified with reference to the previous embodiment is assigned the same reference numerals. In this embodiment, the upper wall 10 is longer and has an upwardly depending lip 18 which functions in use to deflect abdominal tissue away from the opened incision.

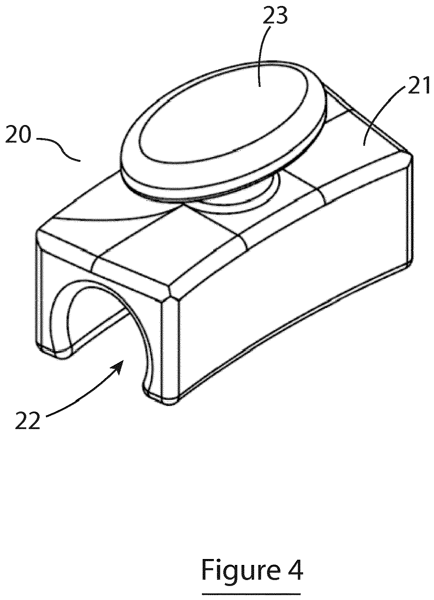

Referring to Figure 4, a coupling element 20 for retro-fitting a retractor 3, 17 to the supporting ring 2 is illustrated, and comprises a carriage 21 with a groove 22 having an elliptical profile on a lower surface thereof dimensioned to engage the supporting ring 2 in a tight but sliding arrangement. The use of a groove having an elliptical profile helps prevent rotation of the carriage on the supporting ring. A slot-engaging lug 23 is provided on a top surface and is dimensioned to engage to engage the re-entrant slot 16 of the upper wall 10 of the retractor 3, 17.

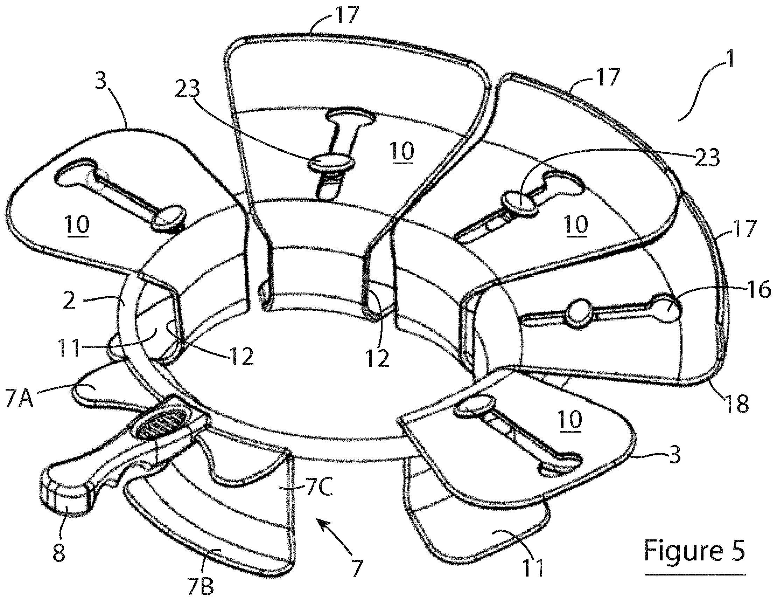

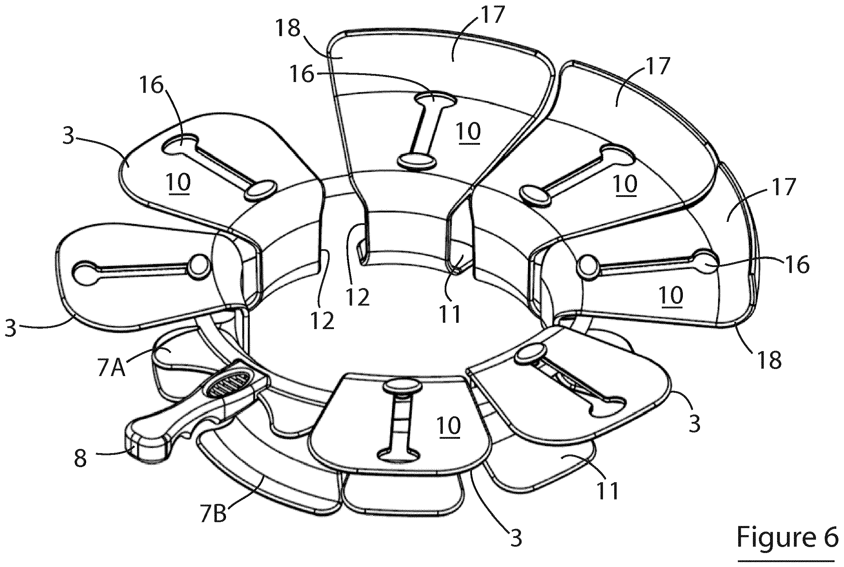

Referring to Figure 5, a surgical system of the invention 1 is illustrated with the retractors 3, 17 attached to the supporting ring 2 in an outwardly facing, and partly retracted, configuration. The modified retractors 17 with the panniculus-deflecting lip 18 are attached to the ring 2 opposite the fixed retractor 7, and the other two retractors 3 are attached to the ring on each side of the fixed retractor. Figures 6 illustrates the same surgical system with two additional retractors 3, and after the retractors 3, 17 have been adjusted radially outwardly to a full retraction position illustrated. It can be seen from Figure 6 how the upper walls of the retractors are dimensioned to dovetail when placed side-by-side on the ring in a fully retracted position.

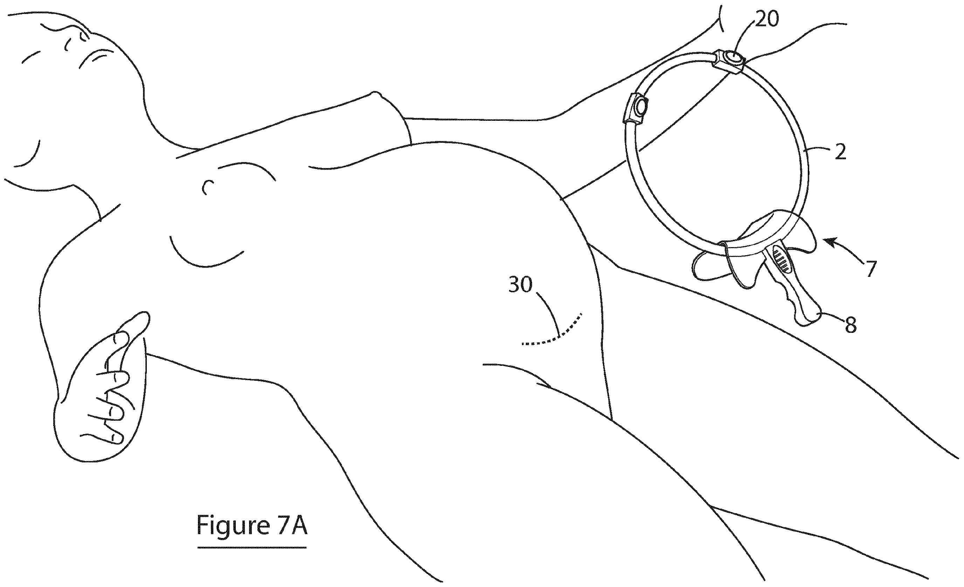

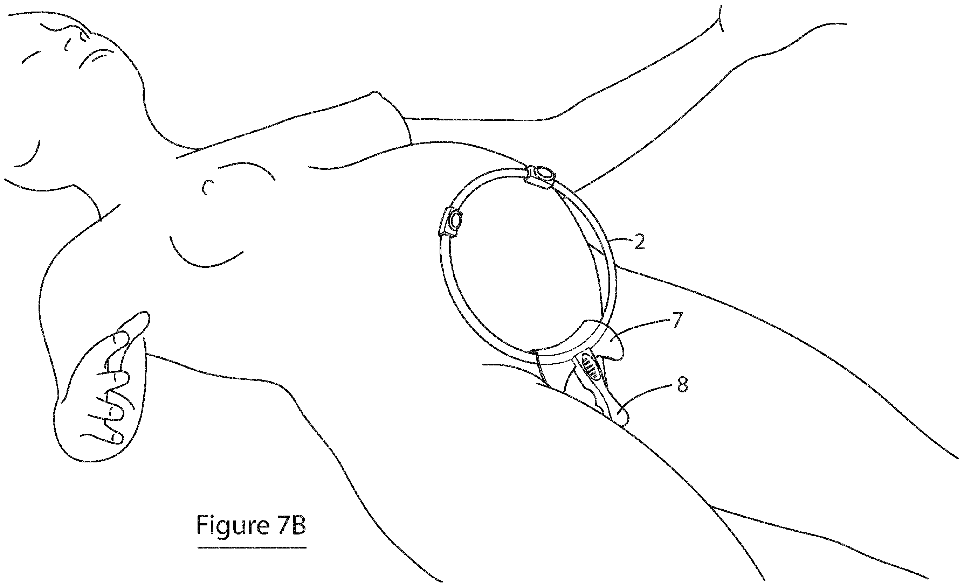

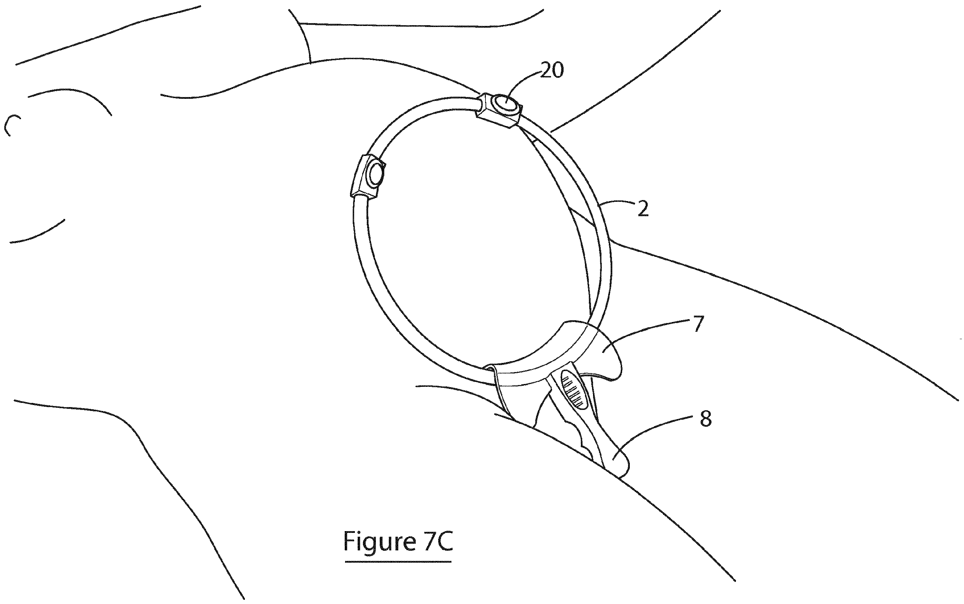

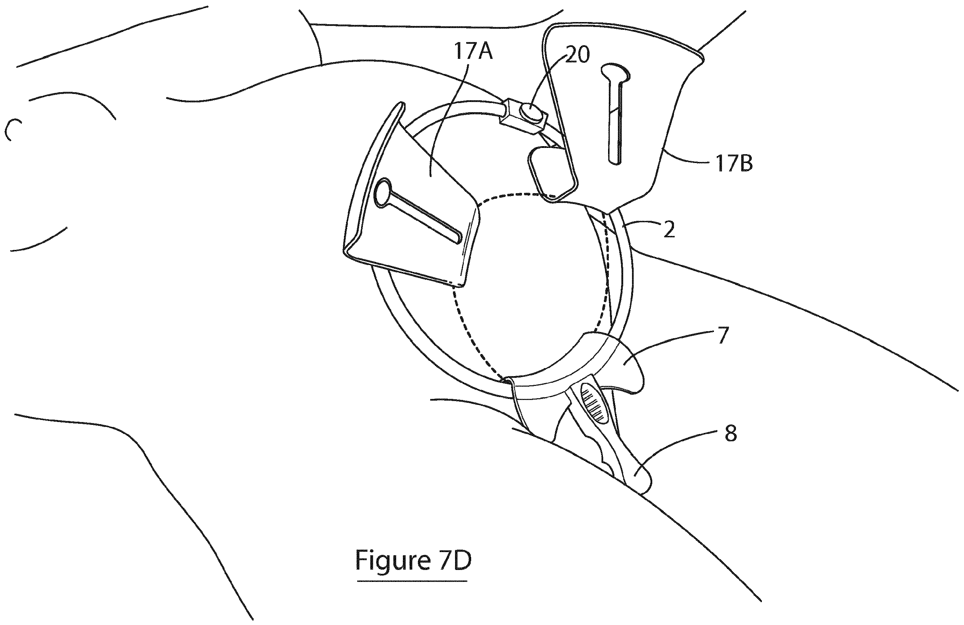

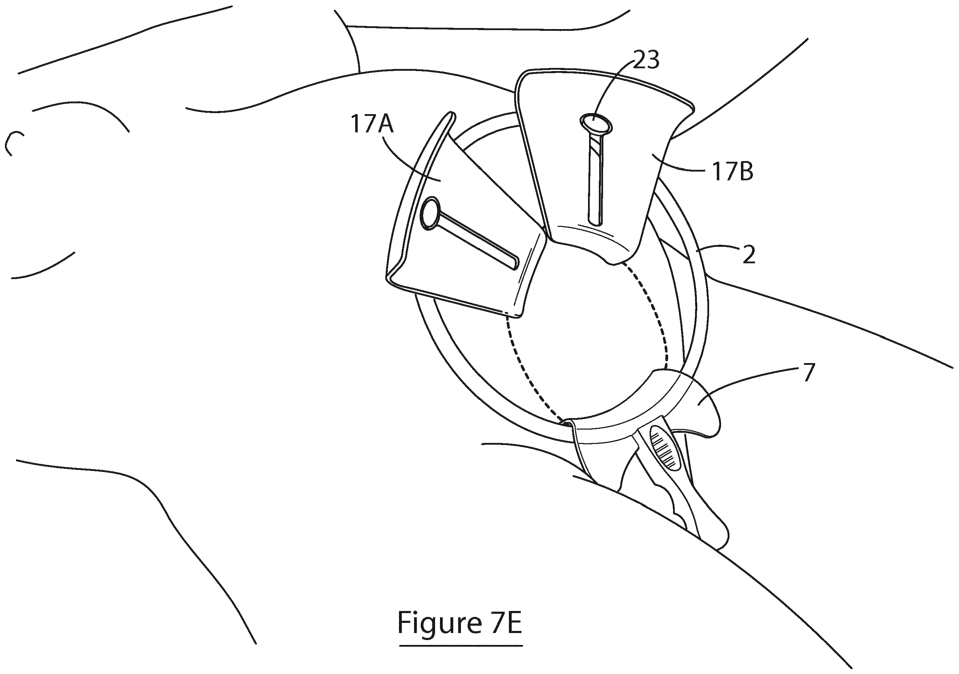

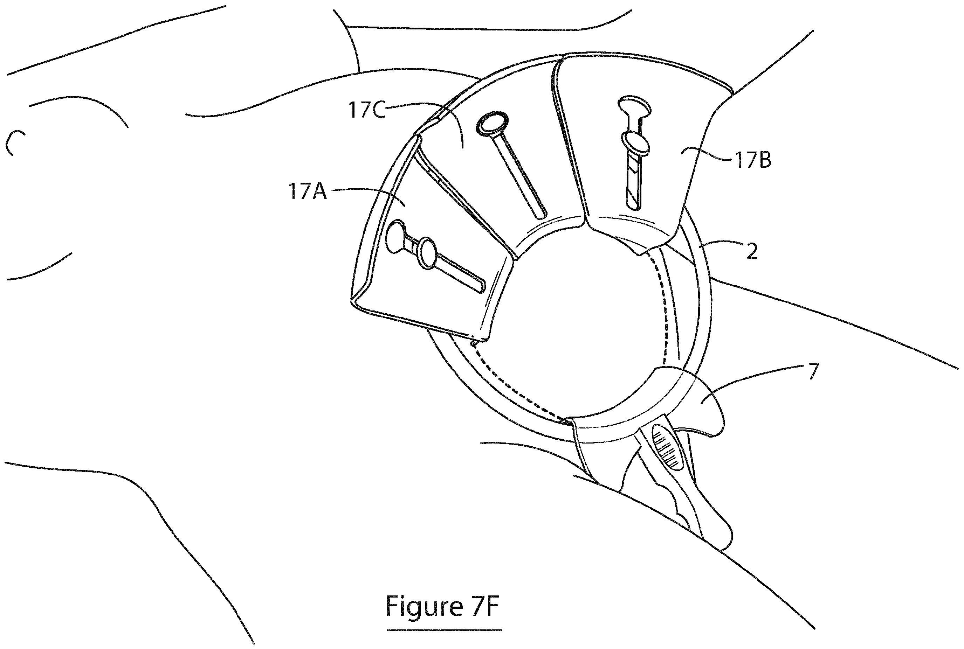

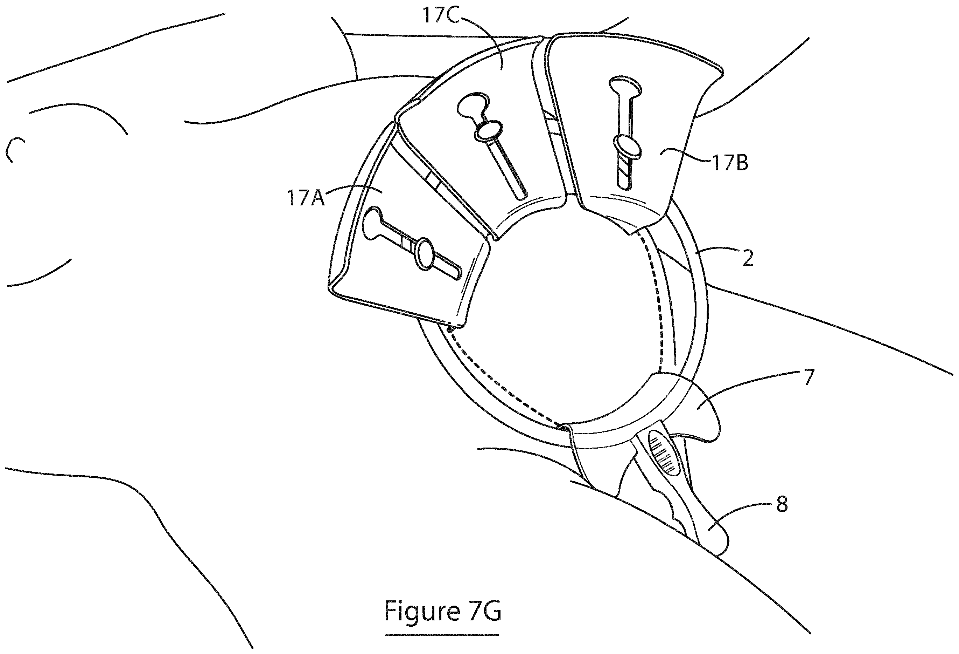

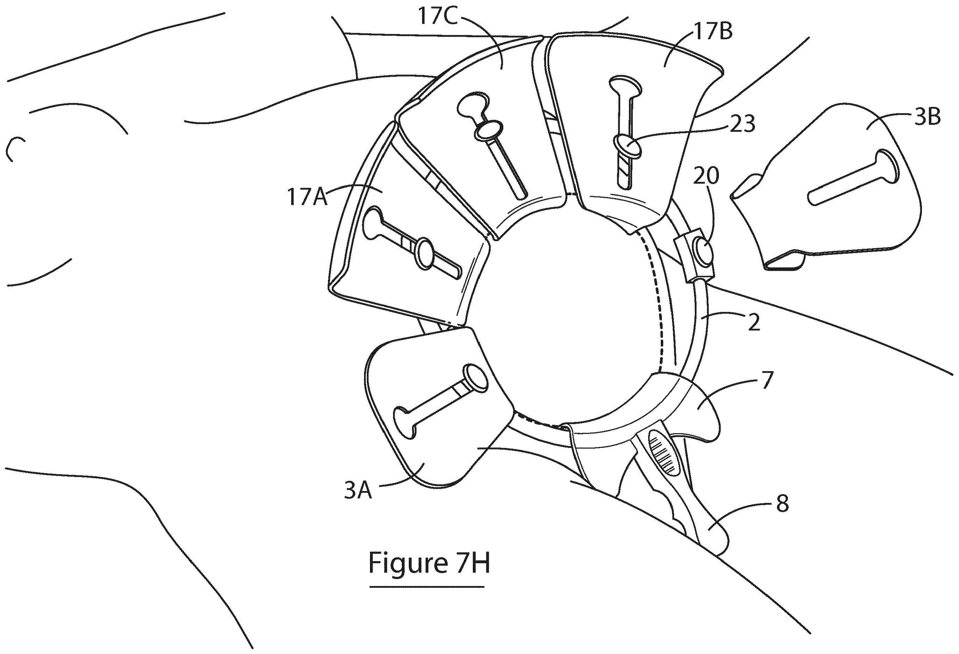

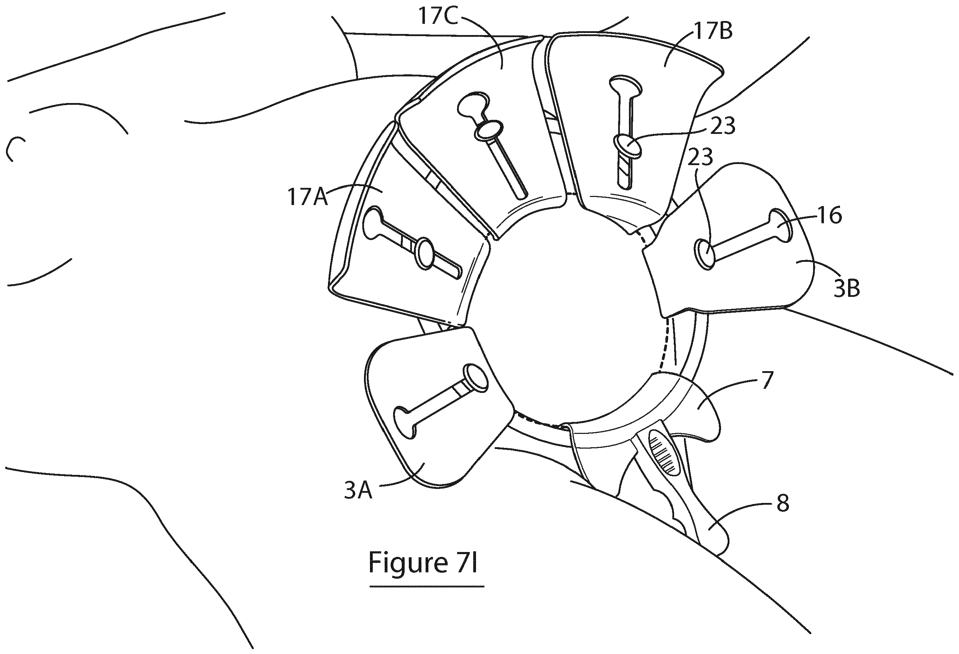

Referring to Figure 7, the use of the surgical access system of the invention in a caesarean section procedure is described, in which parts identified with respect to the previous embodiments are assigned the same reference numerals. It will be appreciated that the following represents one method of the using the system of the invention in surgical access, and that in use some of the steps may be performed in a different order. Also, in the following description, neither the incision, not the manual adjustment of the retractors prior to attachment to the supporting ring is illustrated, although it is described.

In Figure 7A, the supporting ring with integrally formed (fixed) saddle-shaped retractor 7 is presented on the abdomen of a pregnant woman, using the handle 8, and a caesarean incision 30 is shown in dashed lines.

Figure 7B shows the supporting ring 2 after the retractor 7 after it has been inserted into a caesarean incision (dashed line) to cup a section of the lower face of the incision and partially attach the supporting ring to the patient.

Figure 7C shows the supporting ring in place on the woman's abdomen, and the position of the coupling elements 20 at approximately 11 o'clock and 1 o'clock.

Figure 7D shows two adjustable saddle-shaped retractors 17A and 17B, in an outwardly facing position, one of which 17A has been attached to the ring 2, and a second 17B which is about to be attached to the ring. Both retractors comprise a panniculus-deflecting lip 18. Although not illustrated, it will be appreciated that both retractors will at this point have engaged a section of the upper face of the incision, and that the incision will be partially opened (dashed line) following manual movement of the retractor by the surgeon from an initial incision cupping position to a partial retraction position shown in Figure 7D.

Figure 7E shows the two retractors 17A, 17B attached to the supporting ring.

Figure 7F shows a third retractor 17C attached to the supporting ring 2, following circumferential and radial adjustment of the adjacent retractors 17A and 17B, increasing the size of the opened incision. Although not illustrated, the upwardly depending lip 18 of the retractors will deflect the panniculus of the patient away from the opened incision, allowing unfettered access to the opened surgical site.

Figure 7G illustrates further opening of the incision by further radial adjustment and slight circumferential adjustment of the retractors while they are attached to the supporting ring 2.

Figure 7H illustrates the addition of two saddle-shaped retractors 3A, 3B which are inserted into the incision at each side of the incision and manual retraction (not shown) to further open the incision.

Figure 7I shows the retractors 3A, 3B following attachment to the ring 2 and further retraction to fully open the incision allowing the caesarean procedure to proceed.

It will be appreciated from the foregoing description that the use of detachable paddles, that can be manually employed to cup a section of an incision with partially retraction, attachment to the supporting ring, and then radially adjusted to a fully retracted position while attached to the supporting ring, is a less labour intensive process requiring less pairs of hands to hold open the incision. Moreover, the use of the system of the invention allows for more controlled and accurate opening of the incision.

In the method described, manual retraction steps are employed where a surgeon will use a retractor (while detached from the ring) to cup a section of the incision, and manually retract the incision to a point where the retractor can be attached to the ring (whereafter it can be further retracted while attached to the ring). It will be appreciated however that the adjustable saddle-shaped retractors may be designed for use in a way that the retractors engage the incision while attached to the ring and are also retracted while attached to the ring.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A surgical access system (1) adapted to facilitate access to a surgical site through an incision in a patient's body by opening the incision, comprising
a supporting ring (2);
at least two adjustable saddle-shaped retractors (3, 17) configured to cup and manually retract a section of tissue at the incision; and
coupling elements (20) configured for retrofitting each of the adjustable saddle-shaped retractors to the supporting ring in a tissue-retracting position.

2. A surgical access system according to Claim 1, in which the coupling elements are self-locking.

3. A surgical access system according to Claim 1 or 2, in which the supporting ring comprises a non-adjustable saddle-shaped retractor (7) fixed to the supporting ring and optionally an outwardly projecting handle (8) fixed to the supporting ring adjacent the fixed saddle-shaped retractor (7)..

4. A surgical access system as claimed in Claim 3, for accessing a surgical site through a Caesarean incision in a woman's abdomen, in which the supporting ring is dimensioned to allow delivery of a neonate through the ring, and in which the non-adjustable saddle-shaped retractor is a pelvic region retractor.

5. A surgical access system according to any preceding Claim, in which the coupling element is configured to allow radial adjustment of the adjustable saddle-shaped retractor (3, 17) on the supporting ring (2) from a first retraction position to a second retraction position.

6. A surgical access system according to Claim 5, in which an upper wall (10) of the adjustable saddle-shaped retractor (3, 17) comprises a radial slot (16), and the coupling element (20) comprises a lug (23) configured for sliding engagement in the slot and self-locking upon release by the user.

7. A surgical access system according to any preceding Claim, in which the coupling element (20) is configured for detachable attachment to the supporting ring (2) at a plurality of different circumferential positions along the ring.

8. A surgical access system according to Claim 7, in which the coupling element (20) comprises a carriage (21) comprising a supporting ring engaging groove.

9. A surgical access system according to any preceding Claim, comprising at least three adjustable saddle-shaped retractors (3, 17) and associated coupling elements (20).

10. A surgical access system according to any preceding Claim, in which at least one of the adjustable saddle-shaped retractors (3, 17) is configured for adjustment of the height of the retractor.

11. A surgical access system according to any preceding Claim, in which the rear wall (12) of the adjustable saddle-shaped retractors (3, 17) has a convex curvature that optionally substantially matches the curvature of the fully opened incision.

12. A surgical access system according to any preceding Claim, in which the upper wall (10) and lower wall (11) of the adjustable saddle-shaped retractors (3, 17) are splayed outwardly.

13. A surgical access system according to any preceding Claim, in which the supporting ring (2) and saddle-shaped retractors (3, 17) are formed from polymer and disposable.

14. A surgical access system according to any preceding Claim, in which a free end of the upper wall (10) of at least one of the adjustable saddle-shaped retractors (3, 17) comprises an upwardly depending panniculus deflecting lip (18).

15. A surgical access system according to any preceding Claim, in which the supporting ring is configured to emit light into the opened incision during use.
